# EUROPEAN PATENT APPLICATION

(11) **EP 3 482 785 A1**
(43) Date of publication of application: **15.05.2019**
(21) Application number: 18204841.3
(22) Date of filing: 07.11.2018
(51) Int. Cl.: A61M 1/16

(54) **SODIUM AND BICARBONATE CONTROL SYSTEM**

(30) Priority: 08.11.2017 US 201762583007 P; 01.10.2018 US 201816148218
(71) Applicant: Medtronic, Inc., Minneapolis, Minnesota 55432 (US)
(72) Inventor: PUDIL, Bryant J., Plymouth, MN Minnesota 55447 (US); GERBER, Martin T., Maple Grove, MN Minnesota 55369 (US); HOBOT, Christopher M., Rogers, MN Minnesota 55374 (US)
(74) Representative: Maschio, Antonio

(57) **Abstract**

The invention relates to systems and methods for performing dialysis with closed-loop control of sodium and bicarbonate concentrations in the dialysate. The system and methods can use one or more conductivity sensors in a dialysate flow path to determine a water addition rate and a bicarbonate addition rate resulting in a dialysate having desired sodium and bicarbonate concentrations.

## Description

### CROSS-REFERENCE TO RELATED APPLICATION

This application claims the benefit of and priority to U.S. Provisional Patent Application No. 62/583,007 filed November 8, 2017, the entire disclosure of which is incorporated by reference herein.

### FIELD OF THE INVENTION

The invention relates to systems and methods for performing dialysis with closed-loop control over sodium and bicarbonate concentrations in the dialysate. The systems and methods can use conductivity sensors in a dialysate flow path to determine a water addition rate and a bicarbonate addition rate resulting in a dialysate having desired sodium and bicarbonate concentrations.

### BACKGROUND

Control over sodium ions and bicarbonate ions is important during dialysis treatment. The sodium concentration in the dialysate must be carefully controlled for patient health. Dialysate bicarbonate concentration can be important for control over patient acid/base homeostasis. Current methods for control over dialysate sodium concentration and dialysate bicarbonate concentration either assume patient sodium, bicarbonate, and urea levels or require measurements of these parameters prior to treatment. Known systems cannot control dialysate sodium and bicarbonate levels based solely on measurements of dialysate conductivity within a dialysate flow path.

There is a need for systems and methods for measuring and controlling sodium and bicarbonate concentrations in the dialysate based on conductivity measurements of the dialysate. The need extends to systems and methods that can control sodium and bicarbonate concentrations without the need to continually measure patient parameters. The need also includes systems and methods that can use conductivity measurements of the dialysate to estimate patient urea level as well as a need for controllers, processors, software and logic for accurately controlling the sodium and bicarbonate levels of the dialysate.

### SUMMARY OF THE INVENTION

The first aspect of the invention relates to a sodium and bicarbonate control system. In any embodiment, the sodium and bicarbonate control system can comprise a first conductivity sensor in a dialysate flow path; the first conductivity sensor positioned downstream of a dialyzer and upstream of a sorbent cartridge containing urease; a water source fluidly connected to the dialysate flow path downstream of the first conductivity sensor; a bicarbonate source fluidly connected to the dialysate flow path downstream of the sorbent cartridge; a second conductivity sensor in the dialysate flow path; the second conductivity sensor positioned downstream of the bicarbonate source; and a processor programmed to set a water addition rate; the water addition rate set based on a sodium prescription and a conductivity measured by the first conductivity sensor; the processor further programmed to set a post-bicarbonate conductivity set point based on the sodium prescription and a bicarbonate prescription; the processor further programmed to control a bicarbonate addition rate to achieve the post-bicarbonate conductivity set point as measured by the second conductivity sensor.

In any embodiment, the water source can be upstream of the sorbent cartridge.

In any embodiment, the water source can be downstream of the sorbent cartridge.

In any embodiment, the water source can be upstream of the bicarbonate source.

In any embodiment, the water source can be downstream of the bicarbonate source.

In any embodiment, the processor can be further programmed to estimate a sodium concentration of a dialysate based on the conductivity measured by the first conductivity sensor.

In any embodiment, the system can comprise a degasser in the dialysate flow path downstream of the dialysate regeneration unit and upstream of the bicarbonate source.

In any embodiment, the system can comprise a third conductivity sensor downstream of the sorbent cartridge and upstream of the bicarbonate source.

In any embodiment, the processor can determine a urea level of a patient based on the conductivity measured by the first conductivity sensor and a conductivity measured by the third conductivity sensor.

In any embodiment, the processor can control the bicarbonate addition rate by controlling a pump positioned between the bicarbonate source and the dialysate flow path.

In any embodiment, the post-bicarbonate conductivity set point can be a post-bicarbonate conductivity profile.

In any embodiment, the post-bicarbonate conductivity set point can be based on a desired dialysate prescription.

In any embodiment, the post-bicarbonate conductivity set point can be set using a lookup table.

In any embodiment, the post-bicarbonate conductivity set point can be set using data from one or more simulations.

The features disclosed as being part of the first aspect of the invention can be in the first aspect of the invention, either alone or in combination.

The second aspect of the invention relates to a method. In any embodiment, the method can comprise the steps of: measuring a first dialysate conductivity of a dialysis session for a patient in a dialysate flow path downstream of a dialyzer and upstream of a sorbent cartridge containing urease; adding water to the dialysate flow path at a water addition rate; the water addition rate set based on a sodium prescription for the patient and the first dialysate conductivity; measuring a second dialysate conductivity downstream of a bicarbonate source; and adding bicarbonate to the dialysate flow path at a bicarbonate addition rate to result in a second dialysate conductivity of a post-bicarbonate conductivity set point; wherein the post-bicarbonate conductivity set point is set based on the sodium prescription and a bicarbonate prescription.

In any embodiment, the step of adding bicarbonate to the dialysate flow path can comprise adding a bicarbonate concentrate from a bicarbonate source to the dialysate flow path.

In any embodiment, the step of adding water to the dialysate flow path can comprise pumping water from a water source to the dialysate flow path.

In any embodiment, the method can be performed by a dialysis system.

In any embodiment, the method can comprise the steps of measuring a third dialysate conductivity downstream of the sorbent cartridge and upstream of a bicarbonate source; and determining a urea level of the patient based on a difference between the first dialysate conductivity and the third dialysate conductivity.

In any embodiment, the method can comprise pumping a dialysate in the dialysate flow path through a degasser positioned downstream of the sorbent cartridge and upstream of the bicarbonate source.

In any embodiment, the sodium prescription can comprise a sodium profile.

In any embodiment, the method can comprise the step of determining a sodium concentration of the dialysate based on the first dialysate conductivity.

In any embodiment, the first and second dialysate conductivities can be measured by conductivity sensors in the dialysate flow path.

The features disclosed as being part of the second aspect of the invention can be in the second aspect of the invention, either alone or in combination.

### BRIEF DESCRIPTION OF THE DRAWINGS

FIG. 1 is a non-limiting embodiment of a sodium and bicarbonate control system for dialysis treatment.
FIG. 2 is a flow chart illustrating the steps of controlling sodium and bicarbonate concentrations in a dialysate.
FIG. 3 is a non-limiting embodiment of a sorbent cartridge with conductivity sensors.
FIG. 4 is a flow chart illustrating the steps of determining a patient urea level from changes in conductivity in a dialysate flow path.
FIG. 5 is a graph showing a correlation between dialysate conductivity and dialysate sodium concentration.
FIG.'s 6A-C illustrate the correlation between the water addition rate and the conductivity at varying sodium levels.
FIG. 7 is a graph showing the achieved dialysate bicarbonate concentration vs. simulations using the described sodium and bicarbonate control system.
FIG. 8 is a graph showing he achieved dialysate bicarbonate concentration vs. simulations using traditional methods.
FIG. 9 shows the correlation between the change in dialysate conductivities with the dialysate urea concentration.
FIG. 10 illustrates the use of conductivity sensors for closed loop control of dialysate bicarbonate and dialysate sodium concentrations.

### DETAILED DESCRIPTION OF THE INVENTION

Unless defined otherwise, all technical and scientific terms used generally have the same meaning as commonly understood by one of ordinary skill in the art.

The articles "a" and "an" are used to refer to one or to over one (*i.e*., to at least one) of the grammatical object of the article. For example, "an element" means one element or over one element.

The term "adding" or to "add" can refer to adding any component to another component. For example, any one or more of an electrolyte, fluid, gas, or mixtures thereof can be added into or to a second fluid, gas, or mixtures thereof. The terms can also refer to adding any component into a system or conduit.

The term "bicarbonate" can refer to either bicarbonate anions or predecessors to bicarbonate anions, such as salts of bicarbonate, e.g. sodium bicarbonate. Predecessors to bicarbonate anions are any substances that can convert to bicarbonate anions based on conditions within a system or within a patient, such as acetate, lactate, and/or citrate.

The term "bicarbonate addition rate" can refer to a flow rate of bicarbonate ions or a bicarbonate solution being added from a source to a target location. In one non-limiting example, the "bicarbonate addition rate" can refer to the flow rate from a bicarbonate source into a dialysate flow path.

A "bicarbonate concentrate" is a solution containing bicarbonate ions at a higher concentration than intended for use in treatment.

A "bicarbonate prescription" is an intended bicarbonate concentration in the dialysate or blood.

The term "bicarbonate source" can refer to a source of bicarbonate ions or bicarbonate predecessors. The bicarbonate can be in acid or basic form, and can include substances that react to form bicarbonate when used in a dialysis system.

A "component" can be any portion of a larger system or a set of similar or dissimlar components. Non-limiting examples of components are cartridges, containers, reservoirs, sensors, modules, and sorbents.

The term "comprising" includes, but is not limited to, whatever follows the word "comprising." Use of the term indicates the listed elements are required or mandatory but that other elements are optional and may be present.

The term "conductivity" refers to the inverse of the electrical resistance of a fluid.

The term "conductivity sensor" refers to any component capable of measuring the electrical conductance or the electrical resistance of a fluid

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "control," "controlling," or "controls" can refer to the ability of one component to direct the actions of a second component.

A "degasser" can refer to a component capable of removing dissolved and undissolved gasses from fluids.

The term "desired dialysate prescription" can refer to any set of fluid parameters or variables, including concentrations of one or more solutes, of a dialysate used in treatment.

The terms "determining" and "determine" can refer to ascertaining or identifying a particular state or desired state. As used in "determining significant parameters," the phrase refers to ascertaining or identifying any parameter. For example, a system or fluid, or any measured variable(s) or feature(s) of a system or a fluid can be determined by obtaining sensor data, retrieving data, performing a calculation, or by any other known method.

The term "dialysate" can describe a fluid into or out of which solutes from a fluid to be dialyzed diffuse through a membrane. A dialysate typically can contain one or more electrolytes close to a physiological concentration of the electrolyte(s) found in blood.

The term "dialysate flow path" can refer to a fluid pathway or passageway that conveys a fluid, such as dialysate and is configured to form at least part of a fluid circuit for peritoneal dialysis, hemodialysis, hemofiltration, hemodiafiltration or ultrafiltration.

A "dialysis session" can be a period of time in which treatment of a patient using any type form, length, or set of parameters for dialysis is being performed.

The term "dialysis system" can refer to a set of components configured to carry out dialysis therapy of any type including peritoneal dialysis, hemodialysis, hemofiltration, hemodiafiltration, or ultrafiltration.

The term "dialyzer" can refer to a cartridge or container with two flow paths separated by semi-permeable membranes. One flow path can be for blood and one flow path can be for dialysate. The membranes can be in hollow fibers, flat sheets, or spiral wound or other conventional forms known to those of skill in the art. Membranes can be selected from any one or combination of materials: polysulfone, polyethersulfone, poly (methyl methacrylate), modified cellulose, or other materials known to those skilled in the art.

The term "downstream" can refer to a position of a first component in a flow path relative to a second component wherein fluid will pass by the second component prior to the first component during normal operation. The first component can be said to be "downstream" of the second component, while the second component is "upstream" of the first component.

The term "estimate" can refer to an approximation of a value for a particular parameter.

The term "fluidly connectable," "fluidly connect," "for fluid connection," and the like, can refer to the ability of providing for the passage of fluid, gas, or a combination thereof, from one point to another point. The two points can be within or between any one or more of compartments, modules, systems, components, and rechargers, all of any type. The connection can optionally be disconnected and then reconnected.

The term 'initiate a dialysis session" or "initiating a dialysis session" can refer to beginning a treatment of a patient by any type of dialysis.

A "lookup table" can be an electronic or non-electronic table correlating the effects of changing a particular variable or variables on an outcome.

The term "measuring" or "to measure" can refer to determining any parameter or variable. The parameter or variable can relate to any state or value of a system, component, fluid, gas, or mixtures of one or more gases or fluids.

A "patient" or "subject" can be a member of any animal species, preferably a mammalian species, optionally a human. The subject can be an apparently healthy individual, an individual suffering from a disease, or an individual being treated for a disease.

The term "post-bicarbonate conductivity set point" can refer to a conductivity of a dialysate, after addition of bicarbonate, having a desired bicarbonate concentration.

The term "post-bicarbonate conductivity profile" can refer to a conductivity measurement of the dialysate as a function of time and/or volumetric flow. The profile can describe conductivity during a therapy. The profile can include any function of time, such as a curve or a line, or a set period of time.

The term "processor" as used herein is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. The terms can include ROM ("read-only memory") and/or RAM ("random-access memory") associated therewith.

The term "profile" can refer to a function that varies with time and/or volumetric flow. The profile can include any one or more of plural rates, start/stop instructions, and length of time. The profile can also refer to a constant or variable rates specific to a particular patient or subject over time and/or volumetric flow. The profile can further include functions such as a curve or a line, or a set period of time.

The term "programmed," when used referring to a processor or computer, can refer to a series of instructions that cause a processor, software, hardware, or computer to perform certain steps.

The term "pump" refers to any device that causes the movement of fluids, gases, or combinations thereof, by applying suction or pressure.

The terms "pumping" or "to pump" can refer to moving a fluid with a pump.

The term "set point" can generally refer to any desired or intended value to which a variable or parameter is targeted.

"Setting," "to set," and the like, can refer to an adjustment of any parameter, component, or algorithm to any particular value or position. The adjustment can include adjustment in any manner such as positioning a component, performing a physical act, or bringing any parameter, computer, algorithm, or computer into a particular state whether implemented by hand, a processor, a computer, or algorithm.

The term "simulation" can refer to a computerized method of conducting dialysis on a hypothetical patient.

A "sodium and bicarbonate control system" can be a system for use in dialysis that can control a sodium concentration and a bicarbonate concentration in a dialysate entering a dialyzer.

The term "sodium concentration of a dialysate" can refer to an amount of sodium ions dissolved in a given volume of dialysate.

A "sodium prescription" can be an intended sodium concentration in the dialysate or blood.

The term "sodium profile" can refer to a concentration of sodium ions in a dialysate as a function of time or volumetric flow.

The terms "sorbent cartridge" and "sorbent container" can refer to a cartridge containing one or more sorbent materials for removing specific solutes from solution, such as urea. The term "sorbent cartridge" does not require the contents in the cartridge be sorbent based, and the contents of the sorbent cartridge can be any contents that can remove waste products from a dialysate. The sorbent cartridge may include any suitable amount of one or more sorbent materials. In certain instances, the term "sorbent cartridge" can refer to a cartridge which includes one or more sorbent materials in addition to one or more other materials capable of removing waste products from dialysate. "Sorbent cartridge" can include configurations where at least some materials in the cartridge do not act by mechanisms of adsorption or absorption. In any embodiment, a system may include a number of separate cartridges which can be physically separated or interconnected wherein such cartridges can be optionally detached and reattached as desired.

The term "upstream" can refer to a position of a first component or first cartridge in a flow path relative to a second component or second cartridge wherein fluid will pass by the first component prior to the second component during normal operation. The first component or first cartridge can be said to be "upstream" of the second component or second cartridge, while the second component or second cartridge is "downstream" of the first component or first cartridge.

The term "urea level of the patient" can refer to the amount of urea within the body of a patient. The urea level can refer to direct measurements of urea, or to measurement of patient blood urea nitrogen, which is a measure of nitrogen in the blood of a patient that comes from urea. The BUN measurement is given in units of mg/dl.

"Urease" is an enzyme that catalyzes the conversion of urea into carbon dioxide and ammonium ions.

The term "water," as used herein, refers to H₂O in a liquid state. "Water" can refer to portable water, drinking water, purified water, deionized water, tap water, or a solution containing one or more solutes dissolved in the water where the concentration of the solutes is known or able to be determined. The water shall not be limited to any specific purity, disinfection time, toxicity, microbial load, or any other indicator of water quality.

The term "water addition rate" can refer to a flow rate of water from a water source into a dialysate flow path.

A "water source" can be any fluid source from which water can be obtained. The source can be any type of reservoir, fluid line, or receptacle. The water from the water source can be water with or without any dissolved solutes, including one or more buffer or ions.

### Closed Loop Control of Dialysate Sodium and Bicarbonate

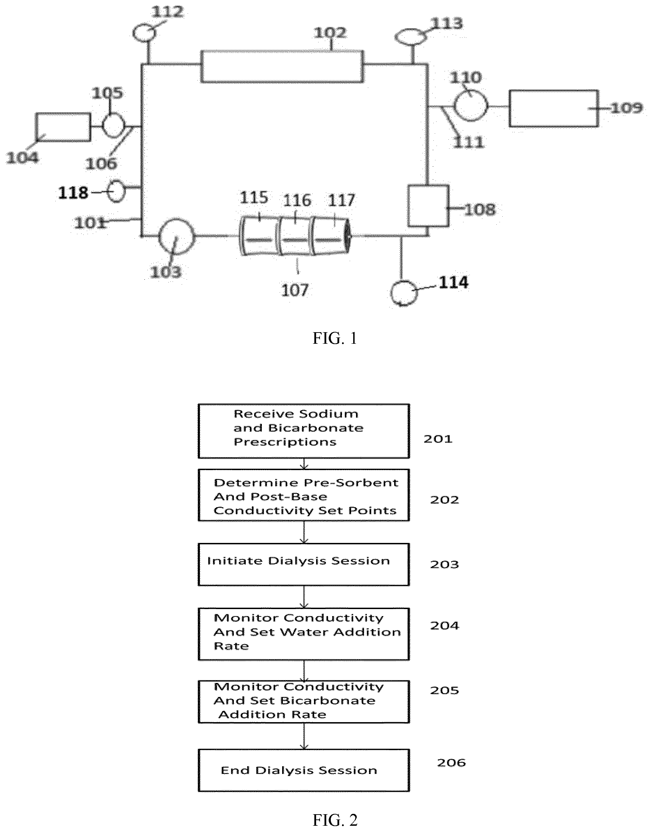

FIG. 1 is a diagram of a sodium and bicarbonate control system for closed loop control of dialysate bicarbonate and sodium concentration. Dialysate in a dialysate flow path 101 can contact blood in an extracorporeal flow path (not shown) across a semipermeable membrane in a dialyzer **102**. Solutes in the blood can cross the semipermeable membrane of the dialyzer **102** into the dialysate in the dialysate flow path **101**. Dialysate pump **103** provides a driving force for moving dialysate through the dialysate flow path **101**. Sorbent cartridge **107** can remove solutes from the dialysate in the dialysate flow path **101**, allowing the dialysate to be reused. The sorbent cartridge **107** can include one or more sorbent modules that can connect together. Alternatively, one or more sorbent modules in sorbent cartridge **107** can be physically separated with fluid lines connecting each module. As illustrated in FIG. 1, the sorbent cartridge **107** can include a first sorbent module **115** containing urease, which catalyzes the conversion of urea to ammonia and carbon dioxide, which further react with water to form ammonium carbonate at physiological pH. The carbon dioxide can be in equilibrium with bicarbonate and carbonate anions, and thus the bicarbonate concentration in the dialysate will depend on the amount of carbon dioxide generated, the temperature, and the pH, and can vary during treatment. Excess carbon dioxide can be removed from the dialysate by degasser **108**. The first sorbent module **115** can optionally include activated carbon. The sorbent cartridge can also include a second sorbent module **116** containing an cation exchange resin, such as zirconium phosphate. A third sorbent module **117** can include an anion exchange resin, such as zirconium oxide. The ammonium ions can be exchanged for hydrogen or sodium ions with the cation exchange resin in the second sorbent module **116**. One of skill in the art will understand that the system can use a single sorbent cartridge containing all sorbent materials, or any number of connected or physically separated sorbent modules. The order of sorbent materials can be changed, so long as a cation exchange resin is present downstream of the urease sorbent material.

Water can be added to the dialysate flow path **101** from water source **104** fluidly connected to dialysate flow path **101**. Water pump **105** pumps water from the water source **104** through conduit **106** and into the dialysate flow path **101**. The water source **104** can optionally include one or more solutes of known concentrations. The system can include the contributions to conductivity of the solutes in setting a conductivity set point as described. Bicarbonate can be added to the dialysate flow path **101** from bicarbonate source **109**, fluidly connected to dialysate flow path **101**. Bicarbonate pump **110** pumps a bicarbonate concentrate from bicarbonate source **109** through conduit **111** and into the dialysate flow path **101**. One of skill in the art will understand that alternative locations for the water source **104** and bicarbonate source **109** can be used. As a non-limiting example, the water source **104** can be moved downstream of the sorbent cartridge **107** yet still be upstream of the first conductivity sensor. The water source **104** can also be upstream or downstream of the bicarbonate source **109**. The bicarbonate source **109** can initially contain a bicarbonate concentrate or a solid bicarbonate source. The solid bicarbonate source can be dissolved with water prior to the dialysis session by pumping water from the dialysate flow path **101** into the bicarbonate source **109** to generate either a saturated bicarbonate solution or a bicarbonate concentrate of known concentration.

Closed loop control over the sodium and bicarbonate concentrations of the dialysate can be achieved with dialyzer outlet conductivity sensor **112** and post-base conductivity sensor **113**. Optionally, the system can include sorbent cartridge outlet conductivity sensor **114**, which can be used to estimate the urea level of the patient as described in conjunction with other sensors and measurements as described herein. A processor (not shown) in communication with the sensors, as well as water pump **105** and bicarbonate pump **110** can control a water addition rate and a bicarbonate addition rate based on data from the conductivity sensors, and a desired dialysate prescription for the patient. The desired dialysate prescription for the patient can include a sodium prescription for the patient, and bicarbonate prescription for the patient. The processor can be programmed to determine a pre-sorbent conductivity set point as measured by dialyzer outlet conductivity sensor **112** based on the sodium prescription, and a post-bicarbonate conductivity set point as measured by post-base conductivity sensor **113** and a bicarbonate prescription. The post-bicarbonate conductivity set point can take into account the sodium concentration of the dialysate as set by the sodium prescription and can be set as a conductivity value to result in a specific bicarbonate concentration in the dialysate after addition of bicarbonate from bicarbonate source **109**. The processor can control water pump **105** to add water to the dialysate flow path **101**, reducing the conductivity of the dialysate until the conductivity of the dialysate as measured by conductivity sensor **118** reaches the pre-sorbent conductivity set point. Dialyzer outlet conductivity sensor **112** can measure the conductivity of the spent dialysate prior to addition of water, and the water addition can be controlled using conductivity sensor **118**. If used with a closed-loop dialysis system, as illustrated in FIG. 1, an equal amount of water can be removed from the dialysate flow path **101** to maintain a constant volume in the dialysate flow path **101** and avoid excess water passing to the extracorporeal flow path (not shown). An ultrafiltration system (not shown) can be included to remove the added water in a "feed and bleed system." The ultrafiltration system can also remove any amount of fluid intended to be removed from the patient during therapy. The processor can account for the dilution water added to the dialysate flow path **101**, as well as any other fluids added to the dialysate flow path **101**, in controlling an amount of fluid bled out of the dialysate flow path **101**. The system can reduce sodium concentration within the controlled volume dialysate flow path **101** by simultaneously operating water pump **105** to add water from water reservoir **104** while simultaneously operating a fluid balance control pump forming part of the ultrafiltration system to remove an equal volume of dialysate by pumping the dialysate to a reservoir or drain (not shown). The processor can control bicarbonate pump **110** to add bicarbonate to the dialysate, raising the conductivity of the dialysate until the conductivity, as measured by post-base conductivity sensor **113**, reaches the post-bicarbonate conductivity set point.

One of skill in the art will understand that the sodium and bicarbonate control system illustrated in FIG. 1 is for illustrative purposes. Additional components and conduits can be added as necessary for dialysis treatment. For example, one or more infusate sources can be included to add potassium, calcium, magnesium, and/or glucose to the dialysate flow path **101**. Additional pumps and reservoirs can be included for ultrafiltration. A heater and temperature sensors can also be included in the dialysate flow path **101**.

FIG. 2 illustrates a flow chart for controlling the sodium and bicarbonate concentrations of the dialysate using the dialysis system of FIG. 1. In step **201** the processor can receive a sodium prescription and a bicarbonate prescription for a patient. In step **202**, the processor can determine a pre-sorbent conductivity set point and a post base conductivity set point. The conductivity set points can include a single value, or can vary throughout the dialysis session as a function of time based on a sodium profile and/or a bicarbonate profile in the sodium prescription and bicarbonate prescription. As described, the processor can determine a pre-sorbent conductivity value that is correlated with a specific sodium concentration of the dialysate. Based on the sodium concentration of the dialysate, the processor can determine a post-base conductivity value that correlates to a specific bicarbonate concentration of the dialysate. The post-bicarbonate conductivity set point can be a post-bicarbonate conductivity profile based on a bicarbonate prescription that changes as a function of time during therapy.

In step **203**, the system can initiate a dialysis session. In step **204**, the system can monitor the conductivity of the dialysate upstream of the sorbent cartridge and control the water pump to add water at a water addition rate, resulting in a pre-sorbent dialysate conductivity equal to the pre-sorbent conductivity set point. In step **205**, the system can monitor the conductivity of the dialysate downstream of the bicarbonate source and add bicarbonate at a bicarbonate addition rate, resulting in a post-bicarbonate conductivity equal to the post-bicarbonate conductivity set point. Steps **204-205** can be continued throughout the dialysis session, optionally with changing conductivity set points in accordance with a sodium and/or bicarbonate prescription. In step **206**, the dialysis session can end.

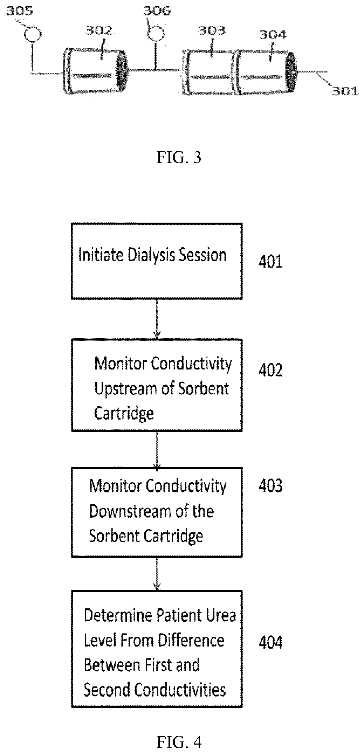

The conductivity sensors can be placed in any location in the dialysate flow path, so long as at least one conductivity sensor is located downstream of the water source, and at least one conductivity sensor is located downstream of the bicarbonate source. FIG. 3 illustrates a non-limiting embodiment of a sorbent cartridge and conductivity sensors. The sorbent cartridge in dialysate flow path **301** can include a first sorbent module **302** containing urease, and optionally activated carbon. A second sorbent module **303** can contain a cation exchange resin, such as zirconium phosphate. A third sorbent module **304** can contain an anion exchange resin, such as zirconium oxide. The order of the second sorbent module **303** and third sorbent module **304** can be changed, or the cation and anion exchange resins can be combined into a single sorbent module. Further, although illustrated as directly connected, the second sorbent module **303** and third sorbent module **304** can be physically separated. A first conductivity sensor **305** can be placed upstream of the first sorbent module **302**. The first conductivity sensor **305** can be used to set the water addition rate as described. A second conductivity sensor **306** can be placed downstream of the first sorbent module **302** and upstream of the second sorbent module **303**. Alternatively, the second conductivity sensor can be placed downstream of the second sorbent module **303** and third sorbent module **304**.

FIG. 4 is a flow chart showing a method of determining a patient urea level based on conductivities measured in a dialysate flow path. In step **401** the dialysis session can be initiated. In step **402**, a first conductivity measurement can be determined upstream of the sorbent cartridge. The first conductivity measurement can be made with a sensor positioned prior to the water source as illustrated by dialyzer outlet conductivity sensor **112** in FIG. 1. Alternatively, the first conductivity measurement can be made just upstream of a urease containing sorbent module, as illustrated by conductivity sensor **305** in FIG. 3. In step **403**, a second conductivity measurement can be made downstream of the sorbent cartridge. The second conductivity measurement can be determined downstream of the sorbent cartridge, as illustrated by sorbent cartridge outlet conductivity sensor **114** in FIG. 1, or just downstream of the urease containing sorbent module, as illustrated by conductivity sensor **306** in FIG. 3. The urea level of the patient can be determined based on the difference between the first and second conductivity measurements, as described.

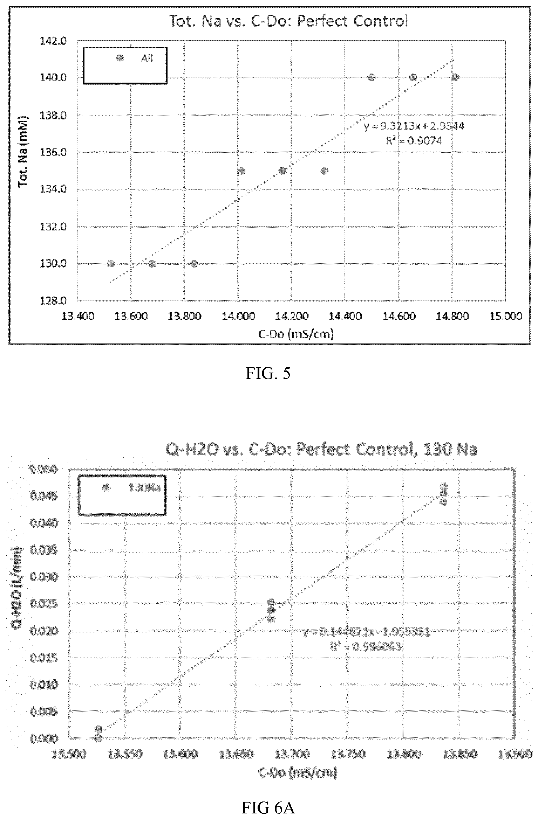

Closed loop control over dialysate sodium concentration is possible because the conductivity of the dialysate is closely correlated to the dialysate sodium concentration. FIG. 5 is a graph showing the correlation. As illustrated in FIG. 5, the spent dialysate conductivity increases linearly with increasing sodium concentration. The conductivities illustrated in FIG. 5 are equivalent to the conductivities as measured by dialyzer outlet conductivity sensor **112** as illustrated in FIG. 1. The three separate plots are from separate sets of simulations. The linear relationship between dialysate conductivity and dialysate sodium concentration exists for a wide range of sodium concentrations, allowing the sodium concentration of the dialysate to be accurately estimated from the conductivity measured by the conductivity sensors.

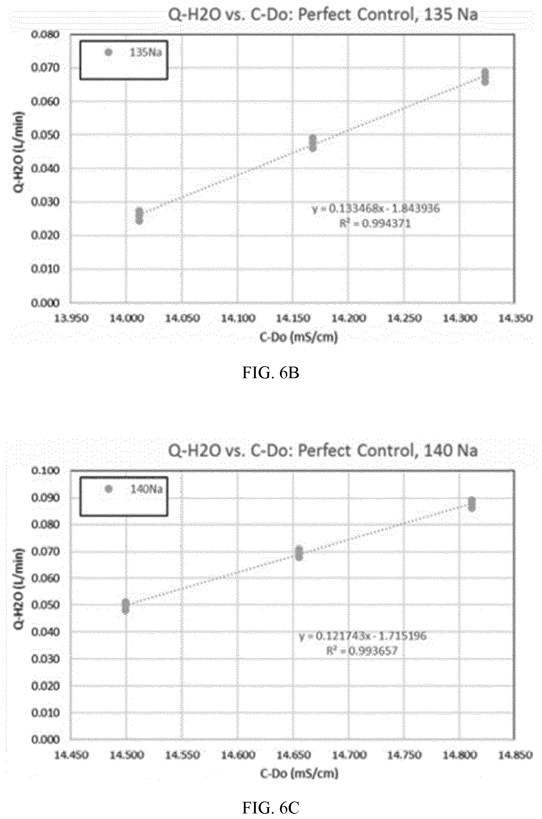

Based on the spent dialysate sodium concentration estimated by the system from the spent dialysate conductivity, the processor can set a water addition rate to achieve a desired sodium prescription. FIG.'s 6A-C illustrate graphs showing the spent dialysate conductivity on the x-axis, and the water addition rate on the y-axis necessary to achieve a dialysate sodium concentration of 140 mM. Each of FIG.'s 6A-C include data from three simulations, shown as the three data points for each conductivity. The correlation between the necessary water addition rate and the dialysate conductivity depends on the dialysate sodium concentration as estimated from the dialysate conductivity. FIG. 6A illustrates the correlation between the water addition rate and the spent conductivity for a dialysate sodium concentration of 130 mM sodium, FIG. 6B illustrates the correlation for a spent dialysate sodium concentration of 135 mM, and FIG. 6C illustrates the correlation for a spent dialysate sodium concentration of 140 mM. Based on the estimated sodium concentration, the processor can select the proper correlation to use, and then set a water addition rate based on the correlation. Each of FIG.'s 6A-C illustrate the water addition rate needed to achieve 140 mM sodium in the dialysate. However, one of skill in the art will understand that similar correlations can be used for any spent dialysate sodium concentration, based on any sodium prescription.

As illustrated in FIG.'s 6A-C, a strong correlation exists between the necessary water addition rate and the spent dialysate conductivity. As such, the processor can use the equations provided in FIG.'s 6A-C to set the water addition rate to achieve the sodium prescription based on the spent dialysate conductivity. The processor can also obtain equations from one or more simulations of dialysis treatment. The simulations can be conducted, and new equations, similar to those in FIG.'s 6A-C can be obtained and used. Alternatively, the processor can use lookup tables including set water addition rates based on the sodium prescription and the spent dialysate conductivity, or any other method of setting a water addition rate known in the art.

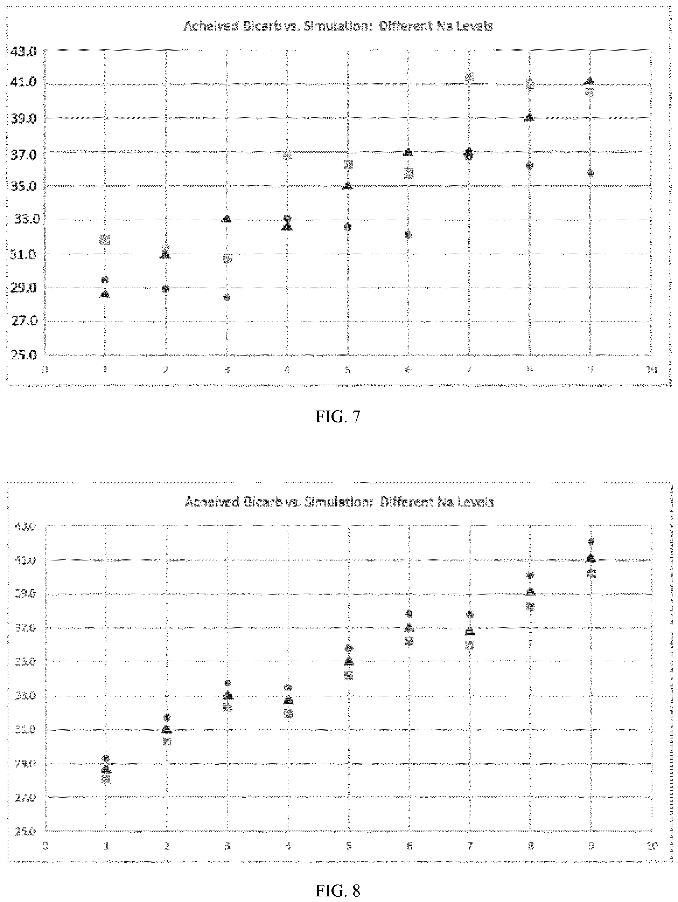

Using the correlations illustrated in FIG.'s 5-6, the system can accurately control the dialysate sodium concentration. For a particular sodium and bicarbonate concentration in the dialysate, there will be a single unique dialysate post-bicarbonate conductivity. The processor can determine the unique conductivity for a given sodium prescription and bicarbonate prescription, and adjust the bicarbonate addition rate until the dialysate conductivity reaches the post-bicarbonate conductivity set point. FIG. 7 illustrates the accuracy of using the two conductivity sensors to control both sodium and bicarbonate. Using the methods described with a bicarbonate prescription of 35 mM, the graph in FIG. 7 shows the actual bicarbonate concentration achieved for differing sodium concentrations. The circles in FIG. 7 represent a sodium level of 130 mM, the triangles represent a sodium level of 135 mM, and the squares represent a sodium level of 140 mM.

The sodium and bicarbonate control system is self-correcting with regards to urea level of the patient. For a given water addition rate, which is based on the conductivity of the spent dialysate, the conductivity of the dialysate exiting the sorbent cartridge will depend on the dialysate urea concentration. A higher urea concentration in the dialysate will result in a higher conductivity because additional sodium and bicarbonate will be formed from the breakdown of urea and exchange of ammonium ions. A lower urea concentration in the dialysate will result in a lower conductivity because less sodium and bicarbonate will be formed from the breakdown of urea and exchange of ammonium ions. The bicarbonate addition rate is self-correcting because with a higher conductivity exiting the sorbent cartridge less bicarbonate needs to be added to reach the post-bicarbonate conductivity set point. With a lower conductivity exiting the sorbent cartridge more bicarbonate needs to be added to reach the post-bicarbonate conductivity set point. Dialysate concentrations of potassium, magnesium, and calcium may have a small effect on the conductivity exiting the sorbent cartridge; however, the levels of potassium, magnesium, and calcium in the dialysate can be estimated fairly accurately based on the dialysate prescription used, and so any error will be small.

FIG. 8 illustrates the accuracy of known bicarbonate control methods. The methods used for the data in FIG. 8 include assuming a patient sodium, urea, and bicarbonate concentration. The circles in FIG. 8 represent a sodium level of 130 mM, the triangles represent a sodium level of 135 mM, and the squares represent a sodium level of 140 mM. Based on the assumptions, water is added to the dialysate to control the conductivity of the dialysate to a target conductivity downstream of the sorbent cartridge. Bicarbonate is then added to the dialysate to control the post-bicarbonate conductivity to a set point. The y-axes in FIG.'s 7-8 are the dialysate bicarbonate concentration, while the x-axes represents arbitrary simulation group numbers. Each data point in FIG.'s 7-8 represents 27 simulations. As an alternative, the sodium and bicarbonate control system can use a measure of patient sodium level to improve accuracy. However, the data in FIG.'s 7-8 use an estimate of dialysate sodium concentration, which is a more important parameter. In any embodiment, knowledge of patient urea, bicarbonate, and sodium levels can be used to improve accuracy. However, each parameter can be estimated as described.

As illustrated in FIG. 7, the methods described result in an average dialysate bicarbonate concentration of 34.8 mM, with a relative standard deviation (RSD) of 11%. The results are comparable with current approaches to dialysate bicarbonate control illustrated in FIG. 8, which result in an average bicarbonate concentration of 35.0 mM and an RSD of 11%. Advantageously, the present methods require no patient modeling, no assumptions as to patient sodium, bicarbonate, and urea concentrations, and are controlled using two conductivity sensors in the dialysate flow path.

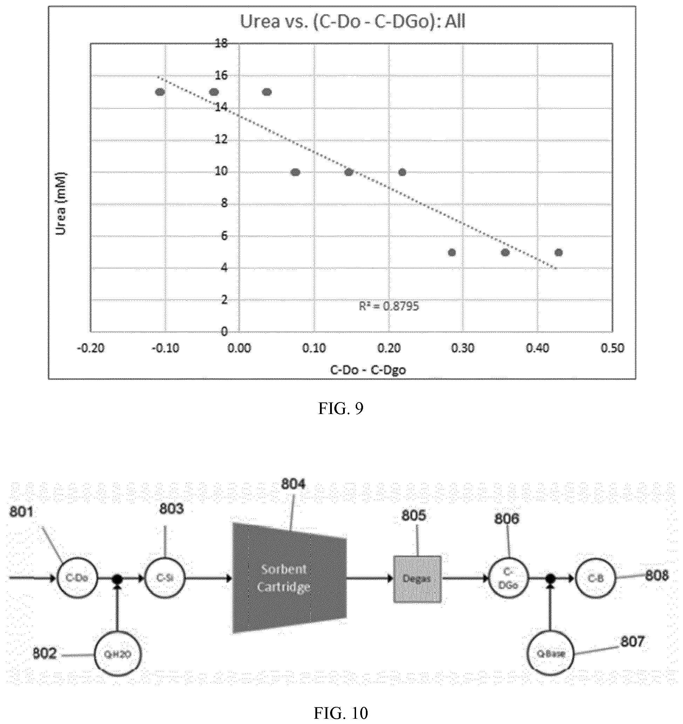

The conductivity sensors described can also estimate the urea level of the patient, as illustrated in FIG. 4. Changes in conductivity as dialysate passes through the sorbent cartridge are correlated to the dialysate urea concentration. For example, the dialysate urea concentration can be estimated using conductivity measurements from dialyzer outlet conductivity sensor **112** and sorbent cartridge outlet conductivity sensor **114** as illustrated in FIG. 1. In certain embodiments, the sorbent cartridge outlet conductivity sensor **114** can instead be placed downstream of the degasser **108** as opposed to upstream of the degasser **108**. FIG. 9 shows the correlation between the difference of the dialysate conductivities measured by a conductivity sensor downstream of the dialyzer and downstream of the degasser with the dialysate urea concentration. The y-axis in FIG. 9 is the urea concentration in the dialysate, while the x-axis is the difference in the two conductivity measurements at a range of spent dialysate urea levels. As illustrated in FIG. 9, a strong correlation exists between the dialysate urea concentration and the change in conductivity. The slope of the line in FIG. 9 provides the equation Urea = -22.484*(C-Do minus C-Dgo) + 13.504, with the units in mM. Based on the urea concentration in the spent dialysate, a person of ordinary skill in the art can determine the urea level of the patient using a simple model of the dialyzer.

Although the data in FIG. 9 was obtained using a first conductivity sensor downstream of the dialyzer and upstream of the water source, one of skill in the art will understand that a similar correlation exists using a first conductivity sensor downstream of the water source.

FIG. 10 illustrates the use of conductivity sensors for closed loop control of dialysate bicarbonate and dialysate sodium concentrations. A first conductivity sensor 801 measures the conductivity of the dialysate downstream of a dialyzer (not shown), which can be used to estimate the dialysate sodium concentration. Based on the conductivity and a sodium prescription, a processor (not shown) determines a water addition rate **802**. Optionally, an additional conductivity sensor **803** measures the dialysate conductivity prior to entering the sorbent cartridge **804**. After passing through the sorbent cartridge **804**, the dialysate passes through degasser **805**. A second optional conductivity sensor **806** can measure the conductivity of the dialysate after passing the degasser **805**. Based on the difference in conductivity as measured by either conductivity sensor **801** or conductivity sensor **803** and conductivity sensor **806**, the processor can estimate the urea level of the patient. The processor also determines a post-bicarbonate dialysate conductivity set point based on the sodium prescription and bicarbonate prescription, and adjusts a bicarbonate addition rate **807** until the dialysate conductivity as measured by conductivity sensor **808** reaches the post-bicarbonate conductivity set point.

One skilled in the art will understand that various combinations and/or modifications and variations can be made in the described systems and methods depending upon the specific needs for operation. Moreover features illustrated or described as being part of an aspect of the invention may be used in the aspect of the invention, either alone or in combination.

The invention may be described by reference to the following numbered paragraphs:
1. A sodium and bicarbonate control system, comprising:
   a first conductivity sensor in a dialysate flow path; the first conductivity sensor positioned downstream of a dialyzer and upstream of a sorbent cartridge containing urease;
   a water source fluidly connected to the dialysate flow path downstream of the first conductivity sensor;
   a bicarbonate source fluidly connected to the dialysate flow path downstream of the sorbent cartridge;
   a second conductivity sensor in the dialysate flow path; the second conductivity sensor positioned downstream of the bicarbonate source; and
   a processor programmed to set a water addition rate; the water addition rate set based on a sodium prescription and a conductivity measured by the first conductivity sensor; the processor further programmed to set a post-bicarbonate conductivity set point based on the sodium prescription and a bicarbonate prescription; the processor further programmed to control a bicarbonate addition rate to achieve the post-bicarbonate conductivity set point as measured by the second conductivity sensor.
2. The sodium and bicarbonate control system of paragraph 1, wherein the water source is upstream of the sorbent cartridge.
3. The sodium and bicarbonate control system of paragraph 1, wherein the water source is downstream of the sorbent cartridge.
4. The sodium and bicarbonate control system of paragraph 1, wherein the water source is upstream of the bicarbonate source.
5. The sodium and bicarbonate control system of paragraph 1, wherein the water source is downstream of the bicarbonate source.
6. The sodium and bicarbonate control system of paragraph 1, the processor further programmed to estimate a sodium concentration of a dialysate based on the conductivity measured by the first conductivity sensor.
7. The sodium and bicarbonate control system of paragraph 1, further comprising a degasser in the dialysate flow path downstream of the sorbent cartridge and upstream of the bicarbonate source.
8. The sodium and bicarbonate control system of paragraph 7, further comprising a third conductivity sensor downstream of the sorbent cartridge and upstream of the bicarbonate source.
9. The sodium and bicarbonate control system of paragraph 8, the processor determining a urea level of a patient based on the conductivity measured by the first conductivity sensor and a conductivity measured by the third conductivity sensor.
10. The sodium and bicarbonate control system of paragraph 1, wherein the processor controls the bicarbonate addition rate by controlling a pump positioned between the bicarbonate source and the dialysate flow path.
11. The sodium and bicarbonate control system of paragraph 1, wherein the post-bicarbonate conductivity set point is a post-bicarbonate conductivity profile.
12. The sodium and bicarbonate control system of paragraph 1, wherein the post-bicarbonate conductivity set point is based on a desired dialysate prescription.
13. The sodium and bicarbonate control system of paragraph 1, wherein the post-bicarbonate conductivity set point is set using a lookup table.
14. The sodium and bicarbonate control system of paragraph 1, wherein the post-bicarbonate conductivity set point is set using data from one or more simulations.
15. A method, comprising the steps of:
   measuring a first dialysate conductivity of a dialysis session for a patient in a dialysate flow path downstream of a dialyzer and upstream of a sorbent cartridge containing urease;
   adding water to the dialysate flow path at a water addition rate; the water addition rate set based on a sodium prescription for the patient and the first dialysate conductivity;
   measuring a second dialysate conductivity downstream of a bicarbonate source; and
   adding bicarbonate to the dialysate flow path at a bicarbonate addition rate to result in a second dialysate conductivity of a post-bicarbonate conductivity set point; wherein the post-bicarbonate conductivity set point is set based on the sodium prescription and a bicarbonate prescription.
16. The method of paragraph 15, wherein the step of adding bicarbonate to the dialysate flow path comprises adding a bicarbonate concentrate from the bicarbonate source to the dialysate flow path.
17. The method of paragraph 15, wherein the step of adding water to the dialysate flow path comprises pumping water from a water source to the dialysate flow path.
18. The method of paragraph 15, wherein the method is performed by a dialysis system.
19. The method of paragraph 15, further comprising the steps of measuring a third dialysate conductivity downstream of the sorbent cartridge and upstream of the bicarbonate source; and determining a urea level of the patient based on a difference between the first dialysate conductivity and the third dialysate conductivity.
20. The method of paragraph 19, further comprising pumping a dialysate in the dialysate flow path through a degasser positioned downstream of the sorbent cartridge and upstream of the bicarbonate source.
21. The method of paragraph 15, wherein the sodium prescription comprises a sodium profile.
22. The method of paragraph 15, further comprising the step of determining a sodium concentration of a dialysate based on the first dialysate conductivity.
23. The method of paragraph 15, wherein the first and second dialysate conductivities are measured by conductivity sensors in the dialysate flow path.

## Claims

1. A sodium and bicarbonate control system, comprising:
a first conductivity sensor in a dialysate flow path; the first conductivity sensor positioned downstream of a dialyzer and upstream of a sorbent cartridge containing urease;
a water source fluidly connected to the dialysate flow path downstream of the first conductivity sensor;
a bicarbonate source fluidly connected to the dialysate flow path downstream of the sorbent cartridge;
a second conductivity sensor in the dialysate flow path; the second conductivity sensor positioned downstream of the bicarbonate source; and
a processor programmed to set a water addition rate; the water addition rate set based on a sodium prescription and a conductivity measured by the first conductivity sensor; the processor further programmed to set a post-bicarbonate conductivity set point based on the sodium prescription and a bicarbonate prescription; the processor further programmed to control a bicarbonate addition rate to achieve the post-bicarbonate conductivity set point as measured by the second conductivity sensor.

2. The sodium and bicarbonate control system of claim 1, wherein the water source is upstream of the sorbent cartridge.

3. The sodium and bicarbonate control system of claim 1, wherein the water source is downstream of the sorbent cartridge.

4. The sodium and bicarbonate control system of any of claims 1 to 3, wherein the water source is upstream of the bicarbonate source.

5. The sodium and bicarbonate control system of any of claims 1 to 3, wherein the water source is downstream of the bicarbonate source.

6. The sodium and bicarbonate control system of any preceding claim, the processor further programmed to estimate a sodium concentration of a dialysate based on the conductivity measured by the first conductivity sensor.

7. The sodium and bicarbonate control system of any preceding claim, further comprising a degasser in the dialysate flow path downstream of the sorbent cartridge and upstream of the bicarbonate source.

8. The sodium and bicarbonate control system of claim 7, further comprising a third conductivity sensor downstream of the sorbent cartridge and upstream of the bicarbonate source.

9. The sodium and bicarbonate control system of claim 8, the processor determining a urea level of the dialysate based on the conductivity measured by the first conductivity sensor and a conductivity measured by the third conductivity sensor.

10. The sodium and bicarbonate control system of any preceding claim, wherein the processor controls the bicarbonate addition rate by controlling a pump positioned between the bicarbonate source and the dialysate flow path.

11. The sodium and bicarbonate control system of any preceding claim, wherein the post-bicarbonate conductivity set point is a post-bicarbonate conductivity profile.

12. The sodium and bicarbonate control system of any of claims 1 to 10, wherein the post-bicarbonate conductivity set point is based on a desired dialysate prescription.

13. The sodium and bicarbonate control system of any of claims 1 to 10, wherein the post-bicarbonate conductivity set point is set using a lookup table.

14. The sodium and bicarbonate control system of any of claims 1 to 10, wherein the post-bicarbonate conductivity set point is set using data from one or more simulations.
